# EUROPÄISCHE PATENTSCHRIFT

(11) **EP 0 418 684 B1**
(45) Veröffentlichungstag und Bekanntmachung des Hinweises auf die Patenterteilung: **16.02.1994**
(21) Anmeldenummer: 90117349.2
(22) Anmeldetag: 08.09.1990
(51) Int. Cl.: C07C 233/38, C07C 233/36, C09J 4/00, A61K 6/00

(54) **Alkandiyl-bis-carbonamide, Adhäsivkomponenten zur Behandlung kollagenhaltiger Materialien zur Anwendung in der Medizin, enthaltend diese Verbindungen**
Alkanediyl-bis-carbonamides, adhesive components containing these compounds for the treatment of collagen based materials for use in medicine
Alkandiyl-bis-carbonamides, composants adhésifs pour le traitement de matériaux à base de collagène contenant ces composés pour l'utilisation dans la médicine

(30) Priorität: 21.09.1989 DE 3931418
(43) Veröffentlichungstag der Anmeldung: 27.03.1991
(73) Patentinhaber: BAYER AG, 51368 Leverkusen (DE)
(72) Erfinder: Müller, Michael, Dr., D-5060 Bergisch Gladbach 2 (DE); Podszun, Wolfgang, Dr., D-5000 Koeln (DE)

(56) Entgegenhaltungen:
- EP-A- 0 141 324
- EP-A- 0 254 950
- DE-A- 2 216 042
- FR-A- 2 001 985
- FR-A- 2 301 539
- US-A- 3 971 765
- ANALYTICAL BIOCHEMISTRY, Band 95, Nr. 1, 1979, S. 260-269, New York/London; R. T. LEE et al: "A simple method for the preparation of polyacrylamide gels containing thioglycoside ligands"

## Beschreibung

Die Erfindung betrifft neue Alkandiyl-bis-carbon-amide (I) und Zubereitungen (11) als Adhäsivkomponente zur Behandlung kollagenhaltiger Materialien zur Anwendung in der Medizin, die Verbindungen (la) enthalten, sowie Verfahren zur Herstellung und die Verwendung der Zubereitungen (11) bei der Herstellung von Mitteln zur Behandlugn kollagenhaltiger Materialien in der Medizin.

Aus FR-A-2 001 985 ist N,N-Trimethylenbisacrylamid und dessen Verwendung bei der Herstellung von transparenten Sicherheitsglasscheiben und anderen Laminatartikeln bekannt geworden.

Die neuen Alkandiyl-bis-carbonamide entsprechen der Formel (I) 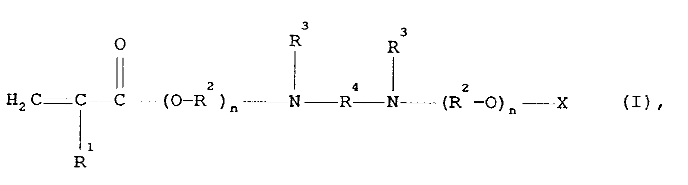 in der
R¹ Wasserstoff oder Methyl bedeutet,
R² und R⁴ zweiwertige aliphatische Reste mit zwei oder drei Kohlenstoffatomen bedeuten, R³ Formyl (-CO-H) bedeutet,
X Wasserstoff oder (Meth)acryloyl 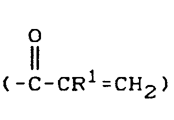 bedeutet und
n für 1 steht.

Die beanspruchten Zubereitungen (II) enthalten Alkandiyl-bis-carbonamide (I a) der Formel 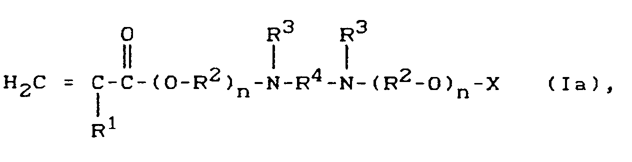 in der
R¹ Wasserstoff oder Methyl bedeutet,
R² und R⁴ zweiwertige aliphatische Reste mit ein bis drei Kohlenstoffatomen bedeuten, R³ Wasserstoff oder Formyl (-CO-H) bedeutet,
X Wasserstoff oder (Meth)acryloyl 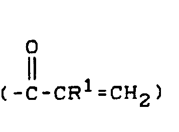 bedeutet und
n für 0 oder 1 steht,
mit der Maßgabe, daß für den Fall R³ gleich H n Null ist und gegebenenfalls Zusätze wie Initiatoren, Lösungsmittel und Füllstoffe.

Kollagenhaltige Materialien sind Gerüsteiweißkörper und Hauptbestandteile der menschlichen und tierischen interzellularen Stützsubstanzen wie Knorpel- und Knochengewebe, Haut und Zahnbein (Dentin). Im Rahmen dervorliegenden Erfindung werden die Adhäsivkomponenten (11) bevorzugt zur Behandlung von Dentin im Zusammenhang mit Zahnreparaturen verwendet.

Besonders im Dentalbereich werden härtende, polymere Materialien als Füllmaterialien bei Zahnreparaturen verwendete. Als härtende, polymere Materialien werden im allgemeinen Füllungen auf Acrylatbasis bevorzugt. Diese polymeren Füllungen haben jedoch den Nachteil, daß sie schlecht am Dentin haften bleiben. Um dieses Problem zu lösen, hat man bisher teilweise Unterschneidungen am Zahnbein vorgenommen; dazu war es erforderlich, über den angegriffenen Bereich hinaus, beachtliche Mengen an frischem Dentin zu entfernen.

Nach einer anderen Methode ätzt man das Dentin und die Schmelzoberfläche mit Säuren, wie z.B. Phosphorsäure, an, und nimmt dann die Füllung vor. Abgesehen davon, daß die Säure eine Reizwirkung im Mundbereich ausübt, dringt sie auch leicht durch die Dentinkanäle in den Zahn und schädigt den Nerv (Pulpa).

In J. Dent. Res. 57, 500-505 (1978), werden aldehydgruppenhaltige Methacrylate der isomeren Hydroxybenzaldehyde beschrieben, die als Grundierungsmittel für Füllungen im Dental bereich verwendet werden können. Die Bindung zwischen Dentin und Füllmasse bleibt jedoch auch nach einer solchen Grundierung unbefriedigend.

In Scand J. Dent. Res. 92, 980-983 (1948) und J. Dent. Res. 63,1087-1089 (1984) werden Grundierungsmittel aus wäßrigem Formaldehyd oder Glutaraldehyd und β-Hydroxyethylmethacrylat (HEMA) beschrieben.

Außerdem sind in der EP-A 0 141 324 Zusammensetzungen aus einem Aldehyd und einem olefinisch ungesättigten Monomer mit aktivem Wasserstoff beschrieben, die eine gute Anbindung an Dentin aufweisen.

Die neuen Zubereitungen (11) auf Basis von Alkandiyl-bis-carbonamiden (I a) bewirken eine starke Verbundhaftung von Materialien, die am Kollagen befestigt werden sollen, z.B. eine Verbundhaftung von Zahnfüllungsmaterial in einer Kavität am Zahn.

N,N'-Methylen- bzw. N,N'-Ethylen-bis-(meth)acrylamide sind bekannte Verbindungen (Röhm GmbH) und wurden zur Immobilisierung von Thioglycosiden (Lee et.al., Anal. Biochem. 95 (1979), 260) und als Bestandteile in Textilausrüstungsmitteln eingesetzt (US 518 779, JP 82/ 95 307, EP 120 316).

Formamidgruppen enthaltende (Meth)-acrylsäureester sind aus der DE-A 2 507 189 bekannt. In der DE-A2 2 507 189 wird auch beschrieben, diese Acrylsäureester als Überzüge oder Klebemittel für Papier und Textilien zu verwenden.

Die Verwendung der erfindungsgemäßen Alkandiyl-bis-carbonamide (la) als Adhäsivkomponente für kollagenhaltige Materialien zur Anwendung in der Medizin war überraschend, weil sie keine reaktiven Gruppen enthalten, die unter milden Bedingungen geeignete chemische Bindungen zu kollagenhaltigen Materialien aufbauen können.

Beispielsweise seien die folgenden erfindungsgemäßen Alkandiyl-bis-carbonamide genannt: 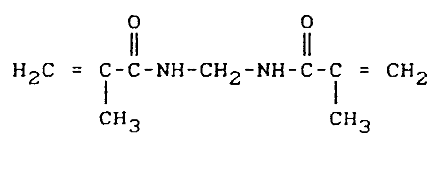 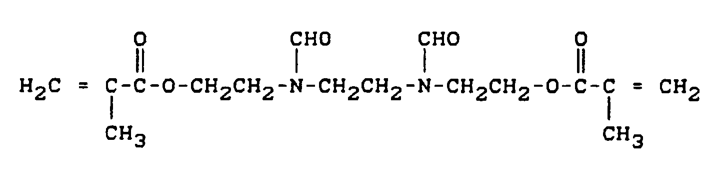 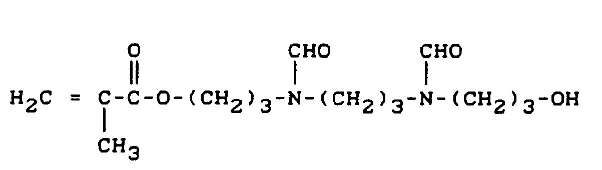 Besonders bevorzugt sind die Alkandiyl-bis-carbonamide der Formeln: 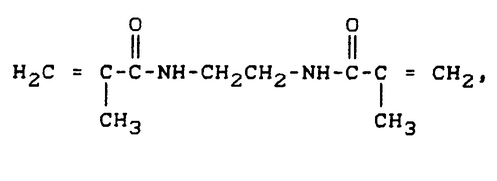 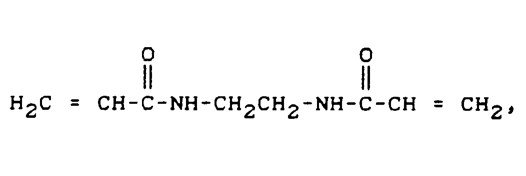 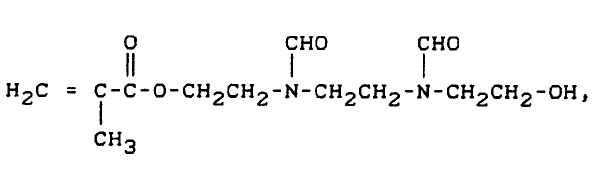 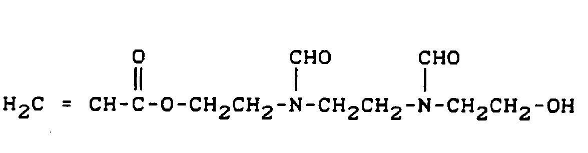 und

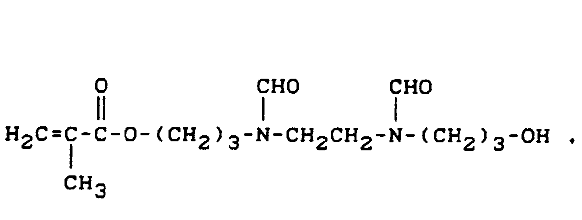

Besonders bevorzugt ist auch der Einsatz von Gemischen dieser erfindungsgemäßen Verbindungen, die neben monofunktionellen (Meth)acrylaten bzw. (Meth)acrylamiden auch bifunktionelle erfindungsgemäße Verbindungen als Vernetzer enthalten.

Die neuen erfindungsgemäßen Alkandiyl-bis-carbonamide (I) können aus Dichloralkanen (III) und Alkanolaminen (IV) hergestellt werden. Die zuerst entstehenden N,N'-Di(hydroxyalkyl)alkylendiamine (V) (DE 2 548 508) lassen sich durch Umsetzung mit Ameisensäureestern (VI) und Veresterung mit einem oder zwei Äquivalenten (Meth) acrylsäure, bzw. deren Ester, Anhydrid oder Säurechlorid (VII), zu den erfindungsgemäßen Produkten (I) umsetzen. 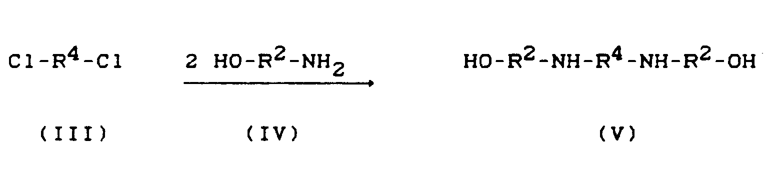 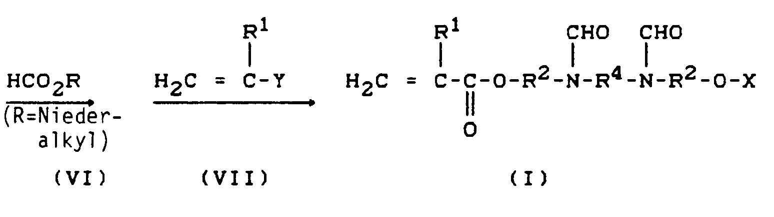 (R¹, R², R⁴ und X haben die Bedeutung wie oben beschrieben, Y = OH, OAlkyl, CI oder O-CR'-CH₂)-Initiatoren im Rahmen der vorliegenden Erfindung sind Radikalbildner, die eine radikalische Polymerisation auslösen. Bevorzugt sind Fotoinitiatoren, die unter der Einwirkung von Licht, beispielsweise UV-Licht, sichtbarem Licht oder Laserlicht, eine radikalische Polymerisation auslösen.

Die sogenannten Fotopolymerisationsinitatoren sind an sich bekannt (Houben-Weyl, Methoden der organischen Chemie, Band E 20, Seite 80 ff, Georg Thieme Verlag Stuttgart 1987). Vorzugsweise handelt es sich um Mono- oder Dicarbonylverbindungen, wie Benzoin und dessen Derivate, insbesondere Benzoinmethylether, Benzil und Benzilderivate, beispielsweise 4,4-Oxidibezil und andere Dicarbonylverbindungen wie Diacetyl, 2,3-Pentandion und a-Diketo-Derivate des Norbornans und substituierter Norbornane, Metallcarbonyle wie Pentacarbonylmangan oder Chinone wie 9,10-Phenanthrenchinon und Naphthochinon. Besonders bevorzugt ist Campherchinon.

Die erfindungsgemäßen Zubereitungen enthalten im allgemeinen 0,01 bis 2 Gew.-Teile, bevorzugt 0,1 bis 0,5 Gew.-Teile des Initiators, bezogen auf 100 Gew.-Teile des eingesetzten Carbonamids. Enthält eines der mit der erfindungsgemäßen Adhäsivkomponente im Kontakt stehenden Fügeteile bereits einen Initiator der beschriebenen Art, kann auf den Initiator in der Adhäsivkomponente auch ganz verzichtet werden.

Die Lösungsmittel im Rahmen der vorliegenden Erfindung sollen die Komponente lösen und sollen, durch die Anwendung bedingt, untoxisch sein. Bevorzugt seien Wasser und flüchtige organische Lösungsmittel wie Methanol, Ethanol, Propanol, Isopropanol, Aceton, Methylethylketon, Essigsäuremethyl-oder-ethylesterund Tetrahydrofuran genannt.

Im allgemeinen setzt man 10 bis 1000 Gew.-Teile, bevorzugt 50 bis 300 Gew.-Teile des Lösungsmittels, bezogen auf das Alkandiyl-bis-carbonamid, ein.

Es kann vorteilhaft sein, den erfindungsgemäßen Zubereitungen Coaktivatoren zuzusetzen, die die Polymerisationsreaktion beschleunigen. Bekannte Beschleuniger sind beispielsweise Amine wie p-Toluidin, Dimethyl-p-toluidin, Trialkylamine wie Trihexylamin, Polyamine wie N,N,N',N'-Tetraalkylalkylendiamin, Barbitursäure und Dialkylbarbitursäure.

Die Coaktivatoren werden im allgemeinen in einer Menge von 0,02 bis 4 Gew.-%, bevorzugt 0,2 bis 1 Gew.- %, bezogen auf die Menge an polymerisierbaren Verbindungen eingesetzt.

Als weitere Komponente können die erfindungsgemäßen Zusammensetzungen Carbonylverbindungen enthalten.

Carbonylverbindungen im Rahmen der vorliegenden Erfindung sind Aldehyde und Ketone, die 1 bis 20, bevorzugt 1 bis 10, und besonders bevorzugt 2 bis 6 Kohlenstoffatome, enthalten. Die Carbonylfunktion kann an einem aliphatischen, aromatischen und heterocyclischen Molekülteil gebunden sein.

Als Aldehyde seien aliphatische Mono- oder Dialdehyde genannt. Bevorzugt wird Formaldehyd, Acetaldehyd, Propionaldehyd, 2-Methylpropionaldehyd, Butyraldehyd, Benzaldehyd, Vanillin, Furfurol, Anisaldehyd, Salicylaldehyd, Glyoxal, Glutardialdehyd und Phthaldialdehyd. Besonders bevorzugt ist der Glutardialdehyd.

Als Ketone seien besonders aliphatische Mono- und Diketone genannt. Bevorzugt sind Butanon, Aceton, Cyclooctanon, Cycloheptanon, Cyclohexanon, Cyclopentanon, Acetophenon, Benzophenon, 1-Phenyl-2-pro- panon, 1,3-Diphenyl-2-propanon, Acetylaceton, 1,2-Cyclohexandion, 1,2-Cyclopentandion und Campherchinon. Besonders bevorzugt ist Cyclopentanon.

Im allgemeinen setzt man 1 bis 1000 Gew.-Teile, bevorzugt 5 bis 50 Gew.-Teile der Carbonylverbindungen, bezogen auf den Carbonamidgruppen enthaltenden (Meth)-acrylsäureester, ein.

Als weitere Komponente können die erfindungsgemäßen Zusammensetzungen (Meth)-acrylsäureester enthalten, die Vernetzungen ausbilden können. (Meth)-acrylsäureester, die Vernetzungen ausbilden können, enthalten im allgemeinen 2 oder mehr polymerisierbare aktive Gruppen im Molekül. Bevorzugt seien Ester der (Meth)-acrylsäure mit 2- bis 5-wertigen Alkoholen mit 2 bis 30 Kohlenstoffatomen genannt. Besonders bevorzugt werden Alkoxy(meth)acrylate und Urethangruppen enthaltende (Meth)acrylate.

Beispielsweise seien (Meth)acrylsäureester der Formel 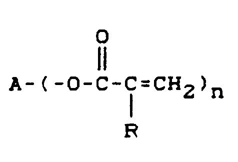 in der
A einen geradkettigen, verzweigten, cyclischen, aliphatischen, aromatischen oder gemischt aliphatischaromatischen Rest mit 2 bis 25 C-Atomen, der durch -O-, NH- oder O-CO-NH-Brücken unterbrochen und mit Hydroxy, Oxy, Carboxy, Amino oder Halogen substituiert sein kann, bedeutet,
R H oder Methyl bedeutet und
n für eine ganze Zahl von 2 bis 8, vorzugsweise 2 bis 4, steht, genannt.

Vorzugsweise seien Verbindungen der folgenden Formeln genannt:

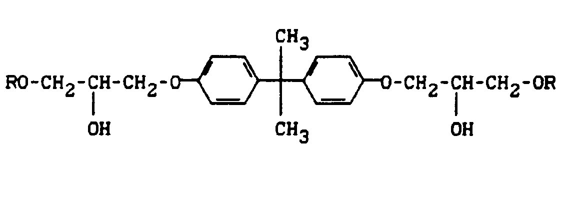

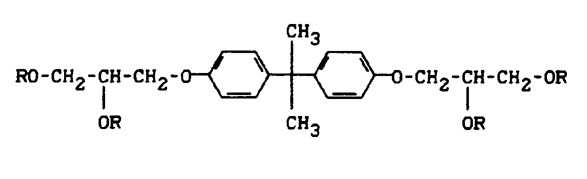

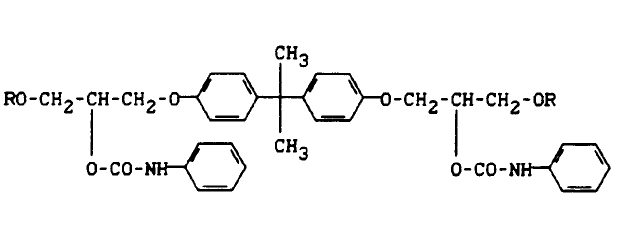

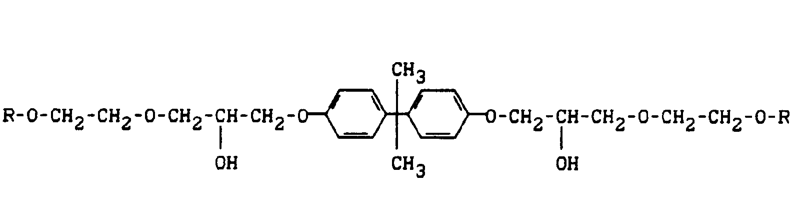

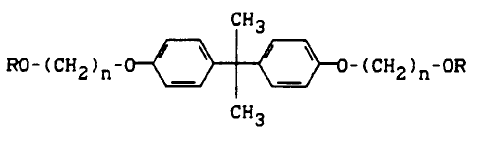

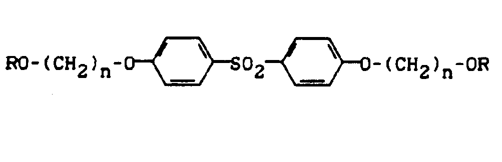

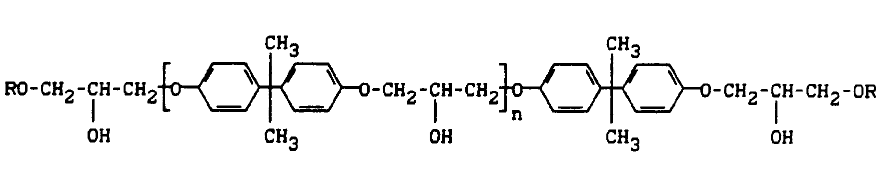

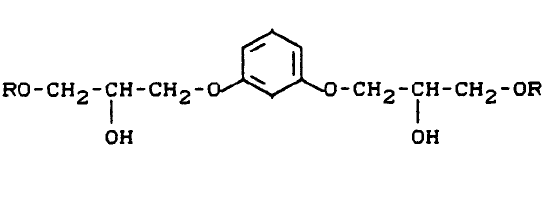

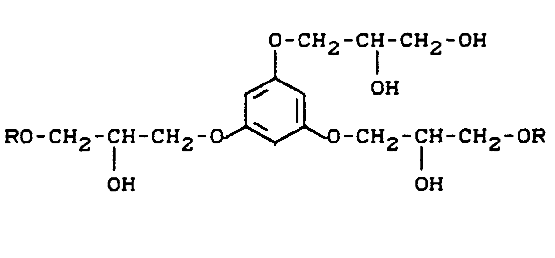

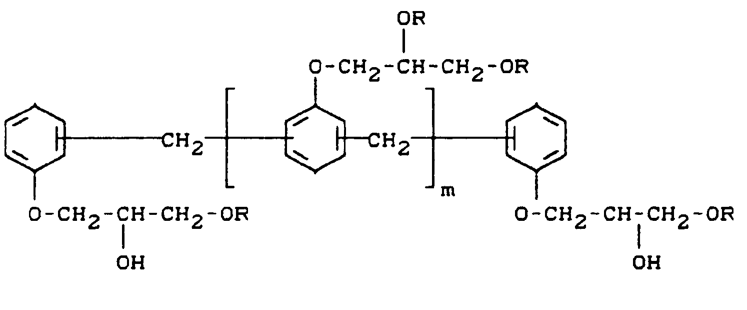

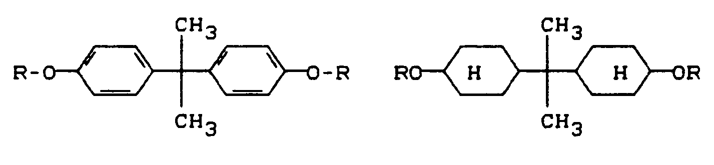

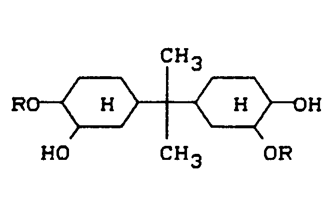

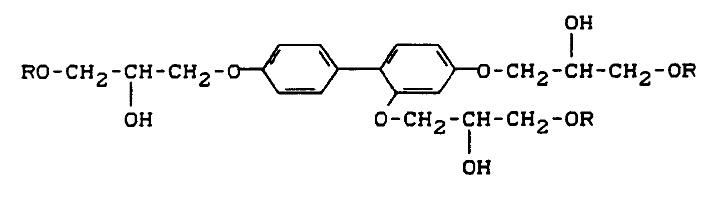

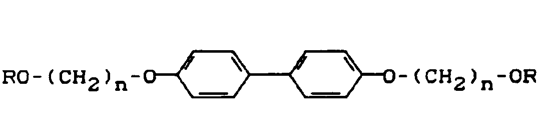

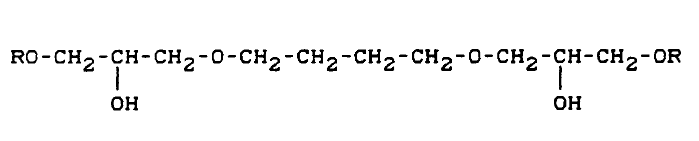

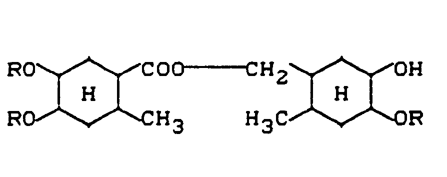

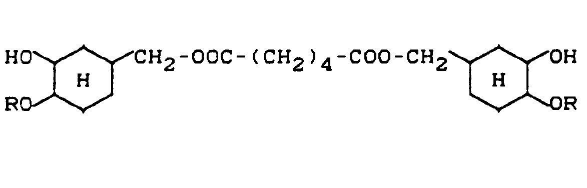

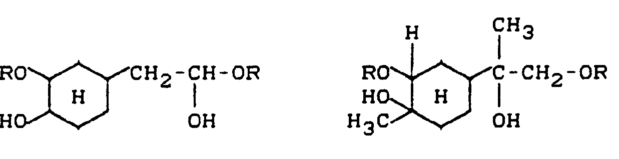

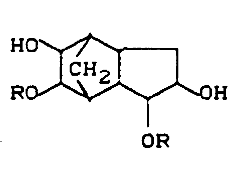

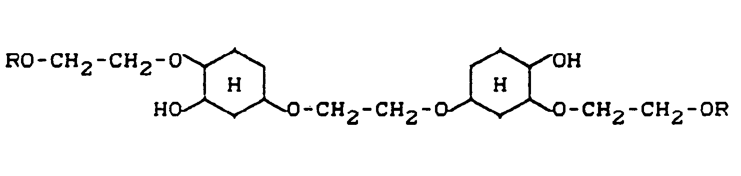

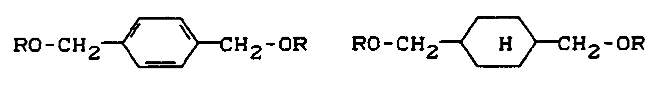

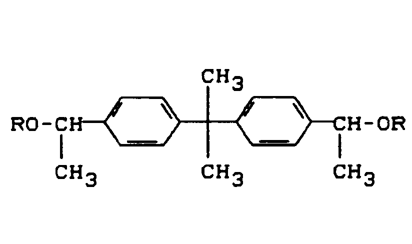

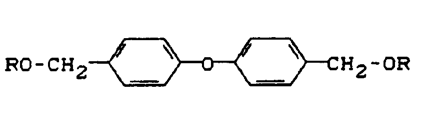

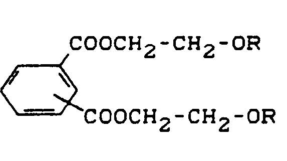

in der ortho, meta- oder para-Form 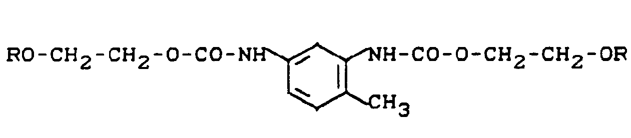 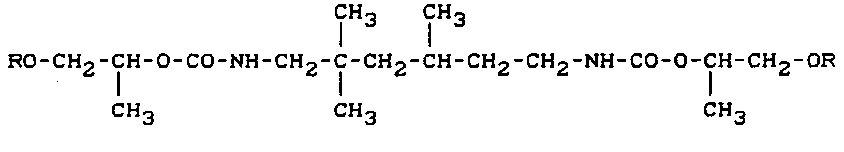 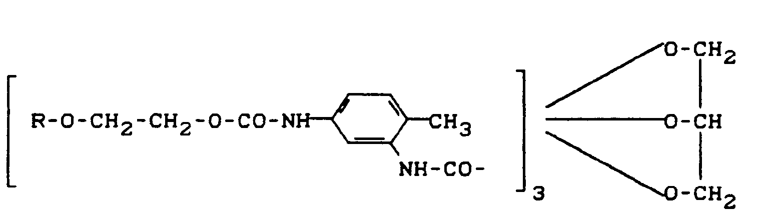 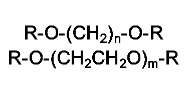 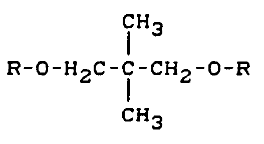 worin
R für 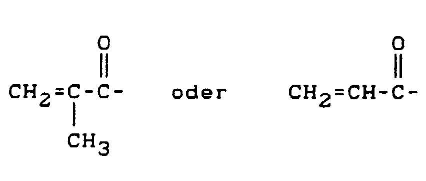 steht,
n eine Zahl von 1 bis 4 und
m eine Zahl von 0 bis 5 bedeutet.

Außerdem seien Derivate des Tricyclodecans (EP-A-00 23 686) und Umsetzungsprodukte aus Polyolen, Diisocyanaten und Hydroxyalkylmethacrylaten (DE-A-37 03 120, DE-A-37 03 080 und DE-A-37 03 130) genannt. Beispielsweise seien die folgenden Monomeren genannt:

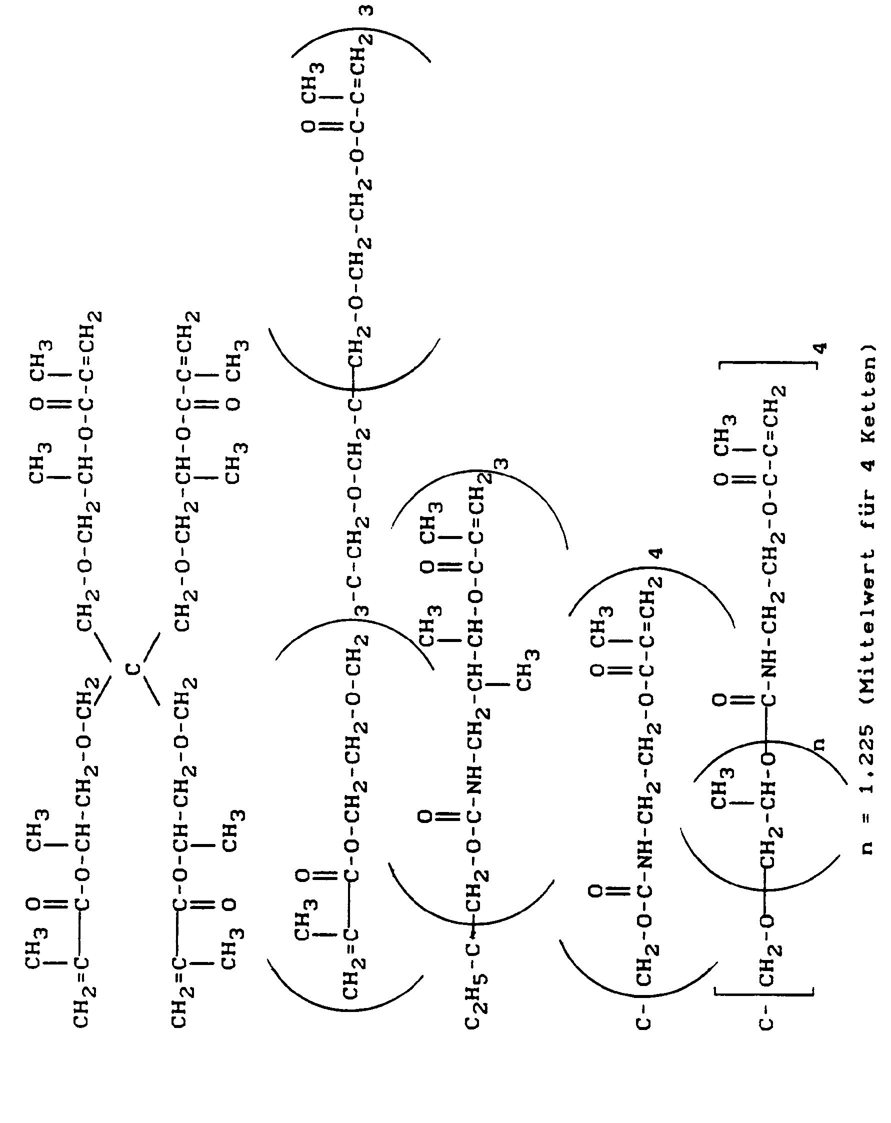

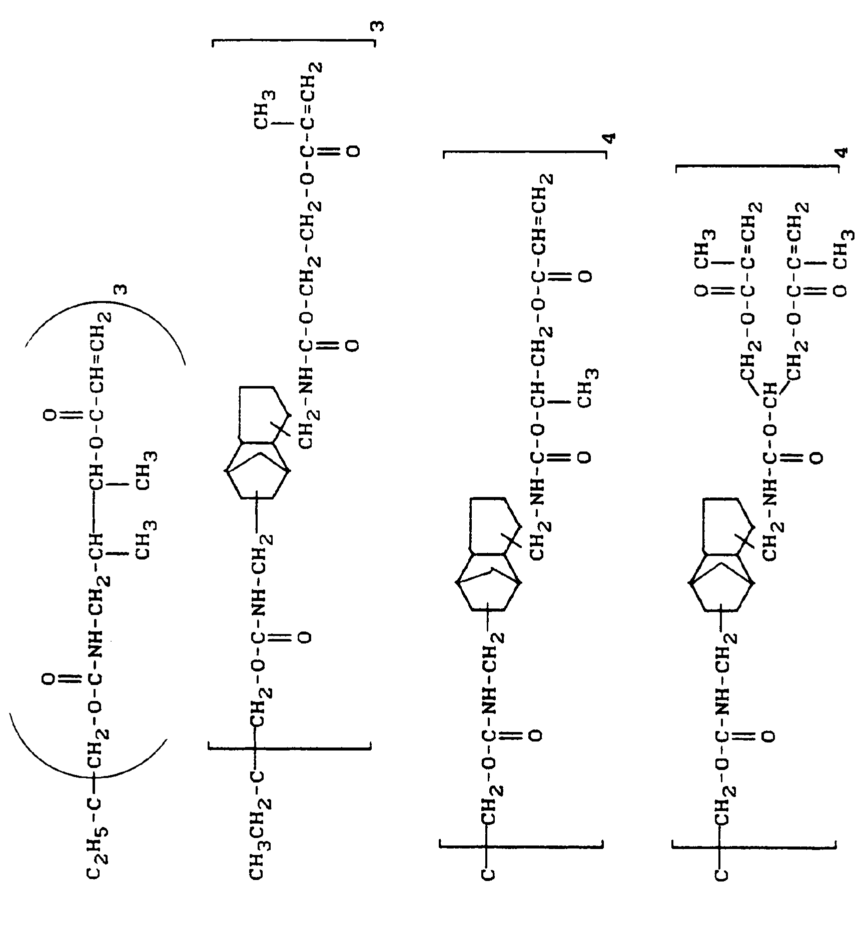

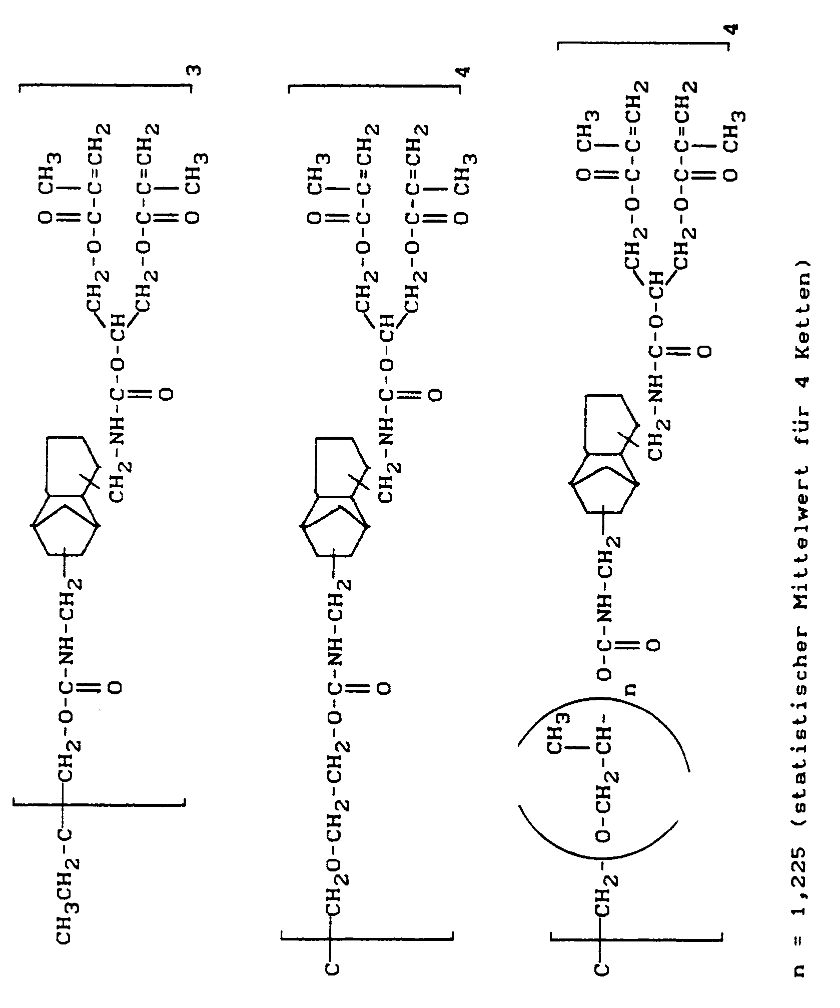

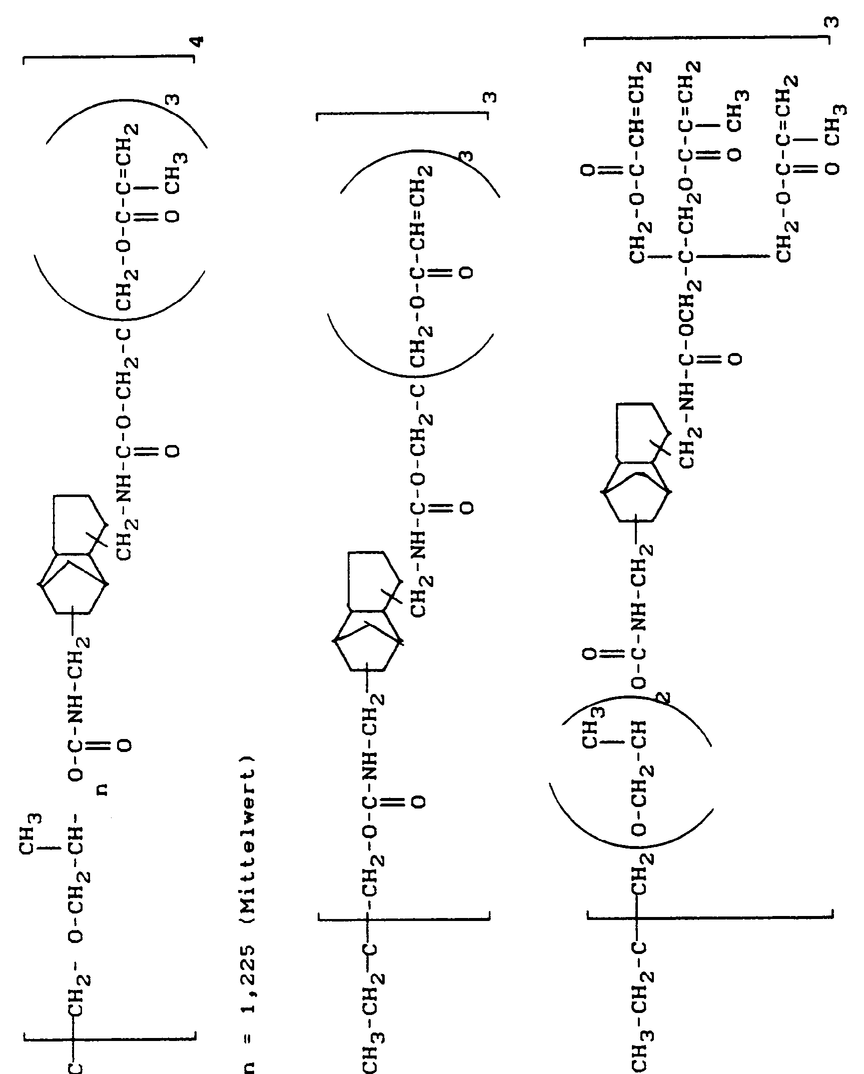

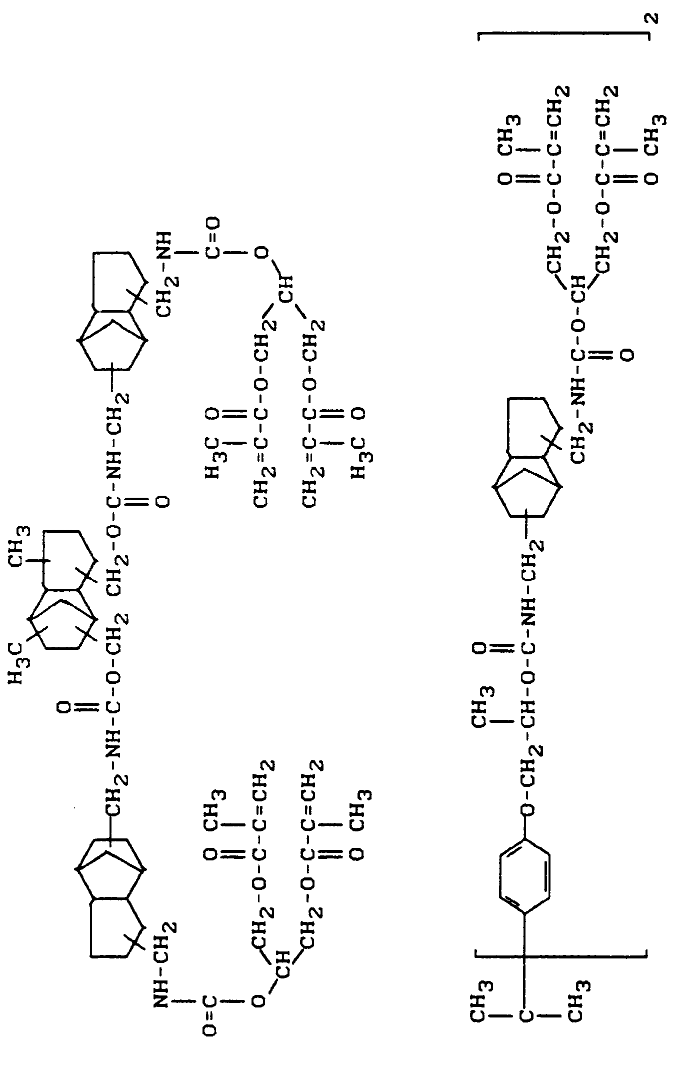

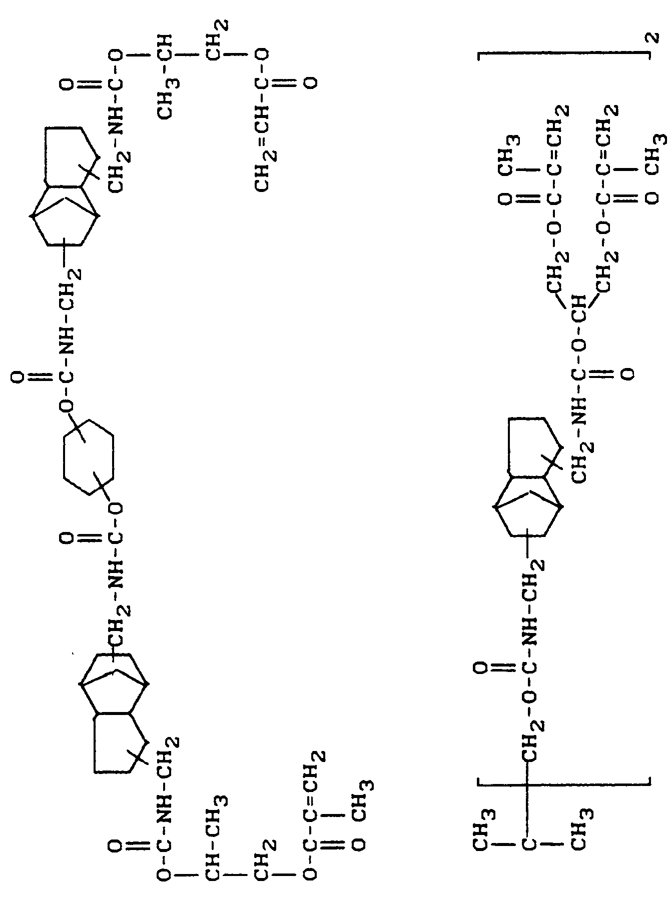

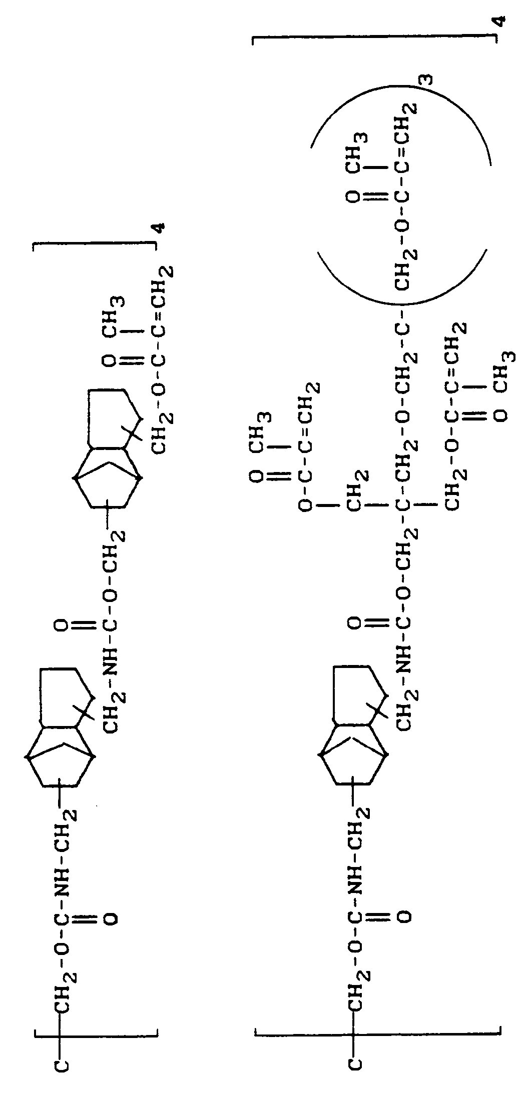

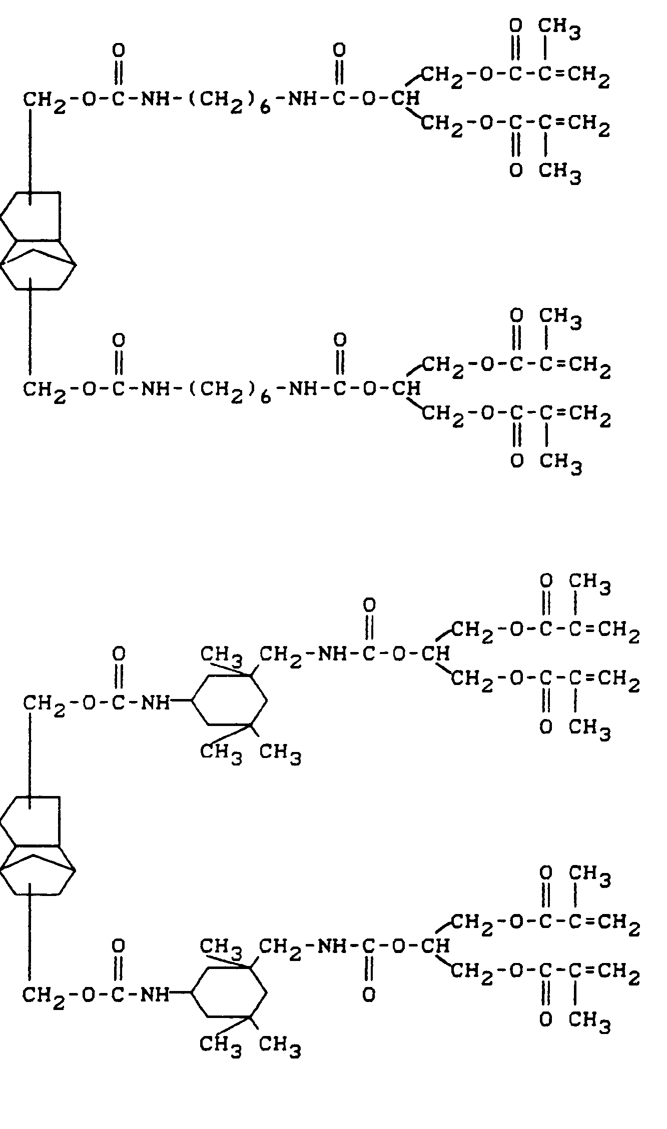

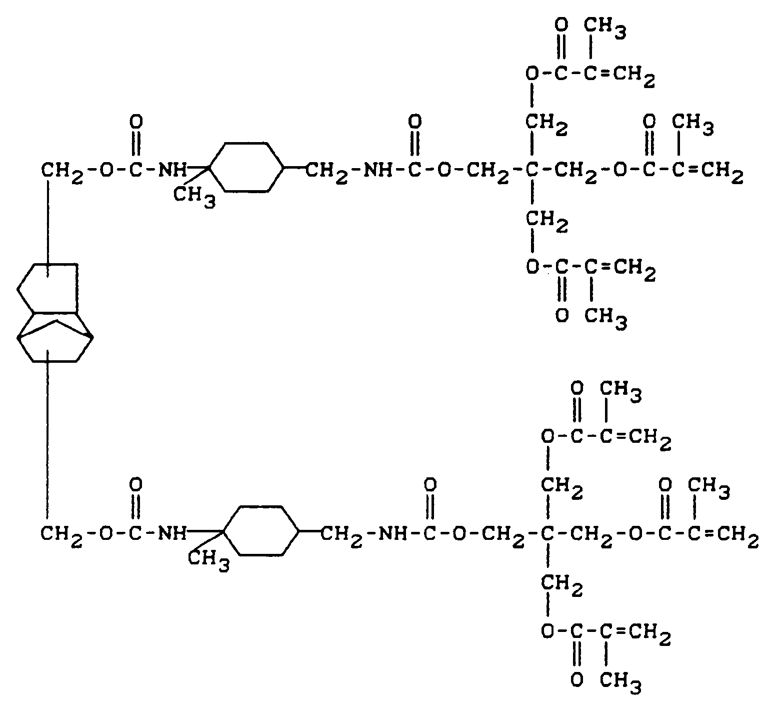

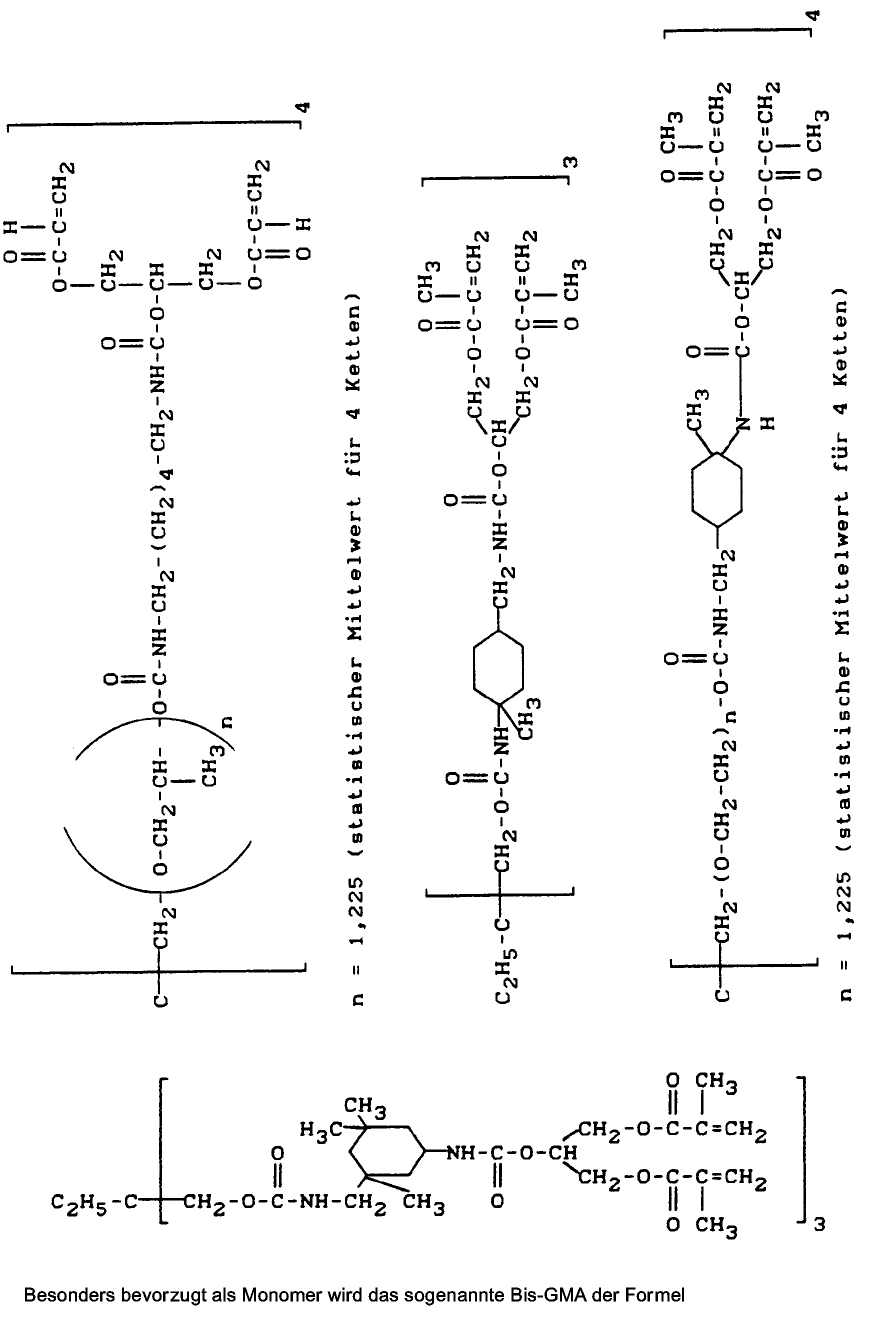

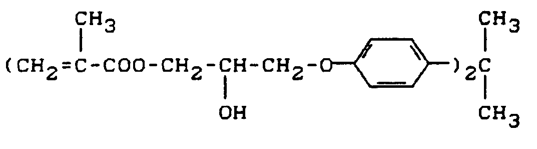

Selbstverständlich ist es möglich, Mischungen der verschiedenen (Meth)-Acrylsäureester, die Vernetzungen ausbilden können, einzusetze. Beispielsweise seien Mischungen von 20 bis 70 Gew.-Teilen Bis-GMA und 30 bis 80 Gew.-Teilen Triethylenglykoldimethacrylat genannt.

Die erfindungsgemäßen Zubereitungen enthalten im allgemeinen 5 bis 80 Gew.-Teile, bevorzugt 10 bis 60 Gew.-Teile Carboxylverbindungen, bezogen auf die Alkandiyl-bis-carbonamide.

Als weitere Komponente können die erfindungsgemäßen Zusammensetzungen Füllstoffe enthalten. Als Füllstoffe werden feine Pulver bevorzugt, die einen Teilchendurchmesser im Bereich von 0,1 bis 100 µm (gegebenenfalls auch in einer polydispersen Verteilung) haben. Füllstoffe können im Dentalbereich übliche Füllstoffe (R. S. Baratz, J. Biomat. Applications, Vol 1, 1987, S 316 ff) wie anorganische Gläser, Siliziumdioxid-, Aluminiumoxid- oder Quarzpulver sein.

Durch einen Anteil an Füllstoffen in den erfindungsgemäßen Zubereitungen entstehen Klebezemente, die besonders zur Befestigung von Brücken-, Kronen- und anderen Verblendmaterialen geeignet sind.

Der Anteil des Füllstoffs beträgt im allgemeinen 20 bis 80 Gew.-Teile, bevorzugt 40 bis 70 Gew.- Teile, bezogen auf die gesamte Zubereitung.

Die Adhäsivkomponenten gemäß dieser Erfindung können weiterhin bis zu 10 Gew.-Teilen üblicher Zusätze wie Stabilisatoren, Inhibitoren, Lichtschutzmittel, Farbstoffe, Pigmente oder Fluoreszenzstoffe enthalten.

Die erfindungsgemäßen Zubereitungen können hergestellt werden, indem man das Alkandiyl-bis-carbonamid und den Initiator und gegebenenfalls die anderen Komponenten durch kräftiges Rühren mischt.

Die Zubereitungen können auch lösungsmittelfrei vorliegen.

Die erfindungsgemäßen Zubereitungen können als Adhäsivkomponente zur Behandlung kollagenhaltiger Materialien zur Anwendung in der Medizin verwendet werden.

In einer besonderen Ausführungsform konditioniert man das kollagenhaltige Material vor der Behandlung mit der erfindungsgemäßen Zubereitung mit einer Flüssigkeit mit einem pH-Wert im Bereich von 0,1 bis 3,5.

Diese enthält im allgemeinen Säuren mit einem pKₛ-Wert kleiner als 5 und gegebenenfalls eine amphotere Aminoverbindung mit einem pKₛ₋Wert im Bereich von 9,0 bis 10,6 und einem pK_{B}-Wert im Bereich von 11,5 bis 12,5. Folgende Säuren können z.B. in der Konditionierflüssigkeit enthalten sein:
Phosphorsäure, Salpetersäure, Brenztraubensäure, Zitronensäure, Oxalsäure, Ethylendiamintetraessigsäure, Essigsäure, Weinsäure, Äpfelsäure, Maleinsäure.

Als amphotere Aminoverbindungen seien vorzugsweise Verbindungen der Formel 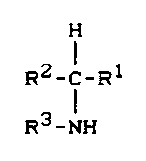 in der
R¹ für eine Carboxylgruppe steht,
R² Wasserstoff, gegebenenfalls durch Hydroxy, Thio, Methylthio, Carboxy, Amino, Phenyl, Hydroxyphenyl oder die Gruppen 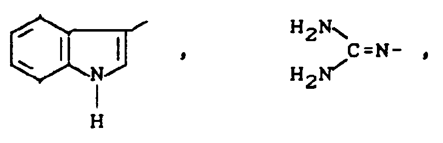 substituierter Niederalkylrest,
R³ Wasserstoff oder Phenyl bedeutet,
wobei die Reste R¹ und R³ durch einen Propylrest verbunden sein können, oder in der
R¹ für Wasserstoff steht,
R² für die Gruppe 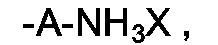 in der
A für einen zweibindigen Alkylenrest mit 1 bis 6 Kohlenstoffatomen und
X für Halogen steht,
   bedeutet und
R³ Wasserstoff bedeutet,
   genannt.

Beispielsweise seien die folgenden amphoteren Aminoverbindungen genannt: Glycin, Serin, Threonin, Cystein, Thyrosin, Asparagin, Glutamin, Alanin, Valin, Leucin, Isoleucin, Prolin, Methionin, Phenylalanin, Tryptophan, Lysin, Arginin, Histidin, N-Phenylglycin, Ethylendiaminhydrochlorid, Ethylendiaminhydrabromid, Propylendiaminhydrochlorid, Propylendiaminhydrobromid, Butylendiaminhydrochlorid, Butylendiaminhydrobromid, Leucinhydrochlorid und Histidinhydrochlorid.

Weiterhin kann die Konditionierflüssigkeit Stoffe aus der Gruppe der Polyethylenglykole und Metallhydroxide enthalten. Insbesondere können die oben aufgeführten mehrbasigen Säuren auch als teilweise Metallsalze eingesetzt werden solange freie Säurefunktionen verbleiben.

Konditionierflüssigkeiten, die mindestens eine der Säuren aus der Gruppe der Brenztraubensäure, Ethylendiamintetraessigsäure und Zitronensäure sowie gegebenenfalls eine amphotere Aminoverbindung aus der Gruppe des Glycins, N-Phenylglycins und Prolins enthalten, sind besonders bevorzugt.

Die Anwendung der erfindungsgemäßen Zubereitungen kann beispielsweise wie folgt durchgeführt werden:
Bei einer Zahnreparatur trägt man beispielsweise nach einer mechanischen Reinigung des kollagenhaltigen Zahnmaterials zuerst die Konditionierflüssigkeit mit etwas Watte auf, läßt eine kurze Zeit (beispielsweise 60 Sekunden) einwirken, spült das Zahnmaterial mit Wasser und trocknet es im Luftstrom. Dann trägt man die erfindungsgemäße Zubereitung beispielsweise mit einem kleinen Pinsel in einer dünnen Schicht auf und trocknet im Luftstrom. Nach der erfindungsgemäßen Behandlung trägt man die eigentliche Füllmasse, beispielsweise im Dentalbereich übliche Kunststoffüllungsmassen (K. Eichner, "Zahnärztliche Werkstoffe und ihre Verarbeitung", Bd 2, S. 135 ff, Hüthig Verlag, 5. Aufl. 1985) auf.

In ähnlicher Weise können die erfindungsgemäßen Zubereitungen zur Befestigung von Kronen, Brücken und ähnlichen Hilfsmitteln verwendet werden.

### Beispiel 1:

Synethese des

Ethandiyl-N-[2-methyl-2-propensäure-ß-(N-formyl)-amino-ethylester] -N'-[ß-hydroxyethyl-(N'-formyl)-amins]

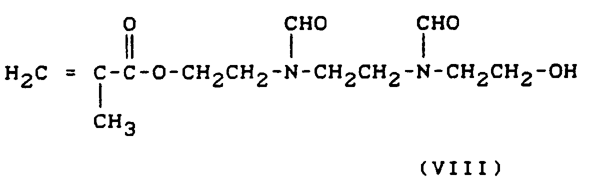

Vorstufe:
Zu 116,16 g (0,750 Mol) Bis-(2-hydroxyethyl)-ethylendiamin in 200 ml Methanol wurden 90,10 g (1.500 Mol) Ameisensäuremethylester getropft und sieben Stunden zum Rückfluß erhitzt. Nach Abziehen aller leicht flüchtigen Bestandteile bei 0,1 Torr und 30°C verbleiben 147,05 g (96 % d.Th.) N,N-Ethandiyl-bis(ß-hydroxyethyl-N-formyl-amin) (IX) in Form eines hellen Öles. 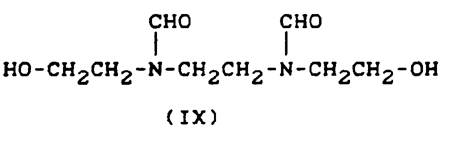 IR (Film): y = 3310, 2902, 1657, 1414, 1398, 1193, 1145, 1060, 858 cm⁻¹. ¹H-NMR (CDCI₃, 200 MHz): 8 = 3.42, 3.56, 3.71
(3 m, zusammen 14 H, CH₂ und OH), 8.02, 8.08
(2 s, zusammen 2 H, CHO in verschiedenen Rotameren) ppm.
MS (70 e V - nach Silylierung):
m/z = 348 (M⁺), 333 (M-CH₃), 188, 146, 144, 126, 116 (CH₂CH₂OTMS⁺) 73 (TMS⁺).

Folgestufe:
Bei -35°C wurden 55,15 g (0,270 Mol) der Vorstufe (IX) in 150 ml trockenem Dichlormethan zusammen mit 37,00 g (0,366 Mol) Triethylamin und 27 mg 2,6-Di-tert.-butyl-4-methylphenol vorgelegt und 28,23 g (0,270 Mol) Methacrylsäurechlorid so zugetropft, daß die Temperatur nicht über -30°C anstieg. Es wurde noch zwei Stunden bei -35°C gerührt, anschließend der ausgefallene Niederschlag bei 0°C abgesaugt und die organische Lösung mehrfach wäßrig extrahiert. Die wäßrige Phase wurde 18 Stunden lang mit Dichlormethan perforiert, die organische Phase getrocknet und zu 30,1 g (41 %. d.Th.) des gewünschten Produkes (VIII) eingeengt, das als leicht gelbliches Öl vorlag.
IR (Film): y = 3400, 2960, 1728, 1670, 1440, 1408, 1320, 1300, 1163, 1078, 950, 818 cm⁻¹.
¹H-NMR (CDCI₃, 200 MHz): δ = 1.93 (bs, 3 H, CH₃), 3.3 - 3,8 (m, 10 H, NCH₂ und CH₂0H), 4.39 (m, 2 H, COCH₂), 5.61, 6.08 (2 m, je 1 H, vinyl.H), 8.00 - 8.10 (m, 2 H, CHO in verschiedenen Rotameren) ppm.

### Beispiel 2:

### Synthese des

N,N'-Ethandiyl-bis [2-methyl-2-propensäure-ß-(N-formyl)-amino-ethylesters]

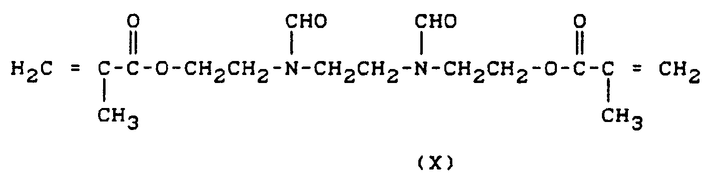

Bei -35°C wurden 51,50 g (0,252 Mol) der Vorstufe (IX) in 200 ml trockenem Dichlormethan zusammen mit 61,00 g (0,603 Mol) Triethylamin und 30 mg 2,6-Di-tert.-butyl-4-methylphenol vorgelegt und über eine Stunde 52,27 g (0,500 Mol) Methacrylsäurechlorid zwischen -30°C und -35°C zugetropft. Es wurde noch zwei Stunden bei -35°C gerührt, der ausgefallene Niederschlag abgetrennt und die organische Phase nach wäßriger Extraktion zu 58,97 g (69 % d.Th.) des gewünschten Produktes (X) eingeengt. Das anfangs gelbliche Öl kristallisierte langsam aus.
Schmelzpunkt: 59 - 60°C

IR (K Br): y = 2960, 1728, 1672, 1432, 1401, 1320, 1298, 1160, 1085, 1031, 948, 813 cm⁻¹. ¹H-NHR (CDCl₃, 200 MHz): δ = 1.93 (bs, 6 H, CH₃), 3.4 - 3.7 (m, 8 H, CH₂N), 4.27 (m, 4 H, CH₂0), 5.62, 6.09 (2 m, je 2 H, vinyl.H), 8.04, 8.09 (2 s, zusammen 2 H, CHO in verschiedenen Rotameren) ppm.

### Beispiele 3 bis 5: Herstellung der Zubereitungen (II)

Die erfindungsgemäßen Klebemittel werden durch intensives Vermischen der in folgenden Beispielen aufgeführten Bestandteile erzeugt.

### Beispiel 3:

14 g Wasser
65 g Aceton
6 g 25 Gew.-%ige, wäßrige Glutardialdehyd-Lösung
15 g N,N'-1,2-Ethandiyl-bis-2-methyl-2-propensäureamid (XI)
40 mg Campherchinon

### Beispiel 4:

18 g Wasser
65 g Aceton
17 g N,N'-1,2-Ethandiyl-bis-2-methyl-2-propensäureamid (XI)
40 mg Campherchinon

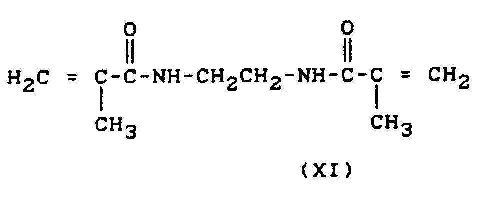

### Beispiel 5:

45 g Wasser
51 g Produkt (VIII)
4 g Produkt (X)
170 mg Campherchinon

### Beispiel 6:

Die Eignung der Klebemittel (11) entsprechend der Beispiele 1 bis 5 wird geprüft, indem die Zugbindungsfestigkeit des lichtaktivierten Kunststoffüllungsmaterials auf Basis mehrfunktioneller Methacrylsäureester und Bariumaluminiumsilikat LUMIFOR Light Curing Composite Universal (U)@, auf Dentin bestimmt wird.

Für die Versuche werden extrahierte menschlische Zähne verwendet, die maximal 3 Monate nach der Extraktion in 1 % Chloraminlösung aufbewahrt wurden. Nachdem diese Zähne sorgfältig unter fließend Wasser gereinigt wurden, erfolgte die Aufbewahrung bis zur Einbettung in Expoxidharz (Lekutherm X 257) in physiologischer Kochsalzlösung.

Unter Verwendung von Schleifpapier unterschiedlicher Körnung wird der Zahn naß angeschliffen bis eine hinreichend große Dentinfläche zur Abbindung eines Kunststoffüllungsmaterialcylinders von 0 3,5 mm freiliegt. Die freigelegte Dentinfläche wurde naß mit Siliziumcarbidpapier 600 abschließend präpariert.

Das Dentin wird nacheinander mit der EDTA-Konditionierfiüssigkeit GLUMA® Cleanser (60 Sekunden Reinigung mit einem Wattepellet, Wasserspülung, Lufttrocknung) und dem Klebemittel (60 Sekunden Einwirkzeit, Lufttrocknung) vorbehandelt.

Zur Herstellung des Probekörpers für den Zugbindungsversuch, wird die wie vor beschrieben präparierte Dentinprobe in ein Stativ mit einer zylindrischen, teilbaren Teflonform gespannt. Diese insgesamt 5 mm hohe Teflonform ist in der oberen Hälfte konisch ausgeführt, so daß mit einem entsprechend geformten Adapter ein Zugversuch durchgeführt werden kann.

Ein Verschlußmaterial auf Basis mehrfunktioneller Methacrylsäureester BAYER Resin L@ wird in dünner Schicht mit einem Pinsel auf die vorbehandelte Dentinoberfläche aufgetragen und mit einem Luftstrom zusätzlich verteilt.

Das Verschlußmaterial wird zunächst im Abstand von 5 mm zur Dentinoberfläche mit einer Polymerisationsleuchte (Translux CL, Fa. Kulzer) bestrahlt. Danach erfolgt ein inkrementales Formfüllen und Lichtaktivieren des Kunststoffüllungsmaterials.

Die Lichtaktivierungszeit für das Kunststoffüllungsmaterial wird aufgrund des großen Volumens mit insgesamt 160 Sekunden angesetzt.

Nach Abschluß der Lichtaktivierung wird der Probekörper entfernt und bis zum Zugversuch im Wasserbad bei 23°C gelagert.

Die Zugbindungsfestigkeit, Kraft beim Bruch der Probe dividiert durch das Kontaktareal zum Dentin wurde mit einer Vorschubgeschwindigkeit von 1 mm/min. gemessen.

Die Bruchfläche am Dentin wird anschließend lichtmikroskopisch zur Beurteilung der Versagenursache überprüft. Hierbei waren vielfach Kohäsivfrakturen zu beobachten, d.h. die mit den erfindungsgemäßen Adhäsivkomponenten erzeugten Verklebungen waren fester als die verklebten Fügeteile selbst. Dies zeigt die gute Leistungsfähigkeit der erfindungsgemäßen Adhäsivkomponenten.

## Patentansprüche (Patentansprüche für folgende(n) Vertragsstaat(en) : AT, BE, CH, DE, DK, FR, GB, IT, LI, LU, NL, SE)

1. Alkandiyl-bis-carbonamide der Formel (I) 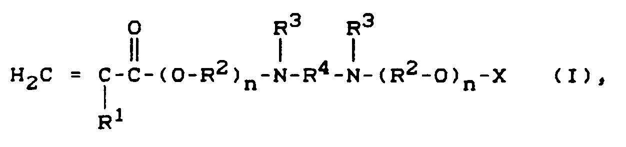 in der
R¹ Wasserstoff oder Methyl bedeutet,
R² und R⁴ zweiwertige aliphatische Reste mit zwei oder drei Kohlenstof fatomen bedeuten, R³ Formyl (-CO-H) bedeutet,
X Wasserstoff oder (Meth)acryloyl 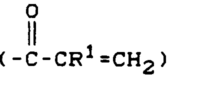 bedeutet und
n für 1 steht.

2. Zubereitungen, enthaltend Alkandiyl-bis-carbonamide der Formel (I a) 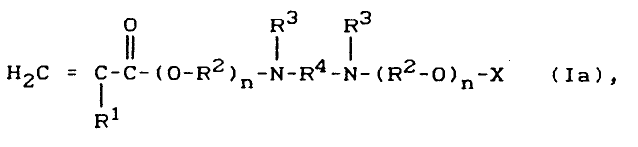 in der
R¹ Wasserstoff oder Methyl bedeutet,
R² und R⁴ zweiwertige aliphatische Reste mit bis zu drei Kohlenstoffatomen bedeuten, R³ Wasserstoff oder Formyl (-CO-H) bedeutet,
X Wasserstoff oder (Meth)acryloyl 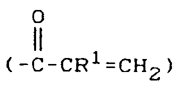 bedeutet und
n für 0 oder 1 steht, mit der Maßgabe, daß für den Fall R³ gleich H, n Null ist und gegebenenfalls Zusätze wie Initiatoren, Lösungsmittel und Füllstoffe als Adhäsivkomponente zur Behandlung kollagenhaltiger Materialien zur Annendung in der Medizin.

3. Zubereitung nach Anspruch 2, enthaltend Alkandiyl-bis-carbonamide (I a), worin
R¹ Wasserstoff oder Methyl bedeutet,
R³ Wasserstoff bedeutet,
R⁴ Ethylen (-CH₂CH₂-) bedeutet,
X Wasserstoff oder (Meth)acryloyl 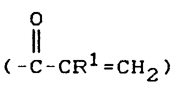 bedeutet und
n für Null steht,

4. Zubereitung nach Anspruch 2, enthaltend Alkandiyl-bis-carbonamide (I a), worin
R¹ Wasserstoff oder Methyl bedeutet,
R² und R⁴ Ethylen (-CH₂CH₂-) bedeuten,
R³ Formyl (-CO-H) bedeutet,
X Wasserstoff oder (Meth)acryloyl 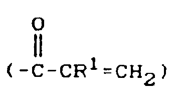 bedeutet und
n für 1 steht.

5. Zubereitung nach den Ansprüchen 2 bis 4, dadurch gekennzeichnet, daß als Initiator ein Radikalbildner aus der Reihe der Mono- oder Dicarbonylverbindungen eingesetzt wird.

6. Zubereitung nach den Ansprüchen 2 bis 5, dadurch gekennzeichnet, daß das Alkandiyl-bis-carbonamid und der Initiator in einem Lösungsmittel gelöst sind.

7. Zubereitung nach den Ansprüchen 2 bis 5, dadurch gekennzeichnet, daß ein Coaktivator zugesetzt wird.

8. Zubereitung nach den Ansprüchen 2 bis 5, dadurch gekennzeichnet, daß als weitere Komponente eine Carbonylverbindung zugesetzt wird.

9. Zubereitung nach den Ansprüchen 2 bis 8, dadurch gekennzeichnet, daß als weitere Komponente (Meth)-acrylsäureester, die Vernetzungen ausbilden können, zugesetzt werden.

10. Zubereitung nach den Ansprüchen 2 bis 9, dadurch gekennzeichnet, daß als weitere Komponente ein Füllstoff zugesetzt wird.

11. Verfahren zur Herstellung von Zubereitungen zur Verwendung als Adhäsivkomponente zur Behandlung kollagenhaltiger Materialien in der Medizin, dadurch gekennzeichnet, daß man Alkandiyl-bis-carbonamide der Formel (I a) 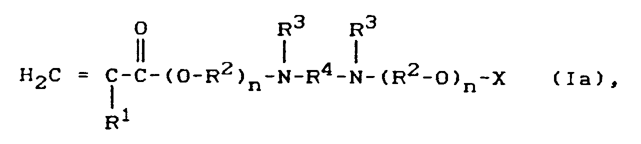 in der
R¹ Wasserstoff oder Methyl bedeutet,
R² und R⁴ zweiwertige aliphatische Reste mit ein bis drei Kohlenstoffatomen bedeuten, R³ Wasserstoff oder Formyl (-CO-H) bedeutet,
X Wasserstoff oder (Meth)acryloyl 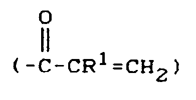 bedeutet und
n für 0 oder 1 steht,
mit der Maßgabe, daß für den Fall R³ gleich H n Null ist und gegebenenfalls Initiatoren in einem Lösungsmittel mischt.

12. Verwendung von Zubereitungen, enthaltend Alkandiyl-bis-carbonamide der Formel (I a) 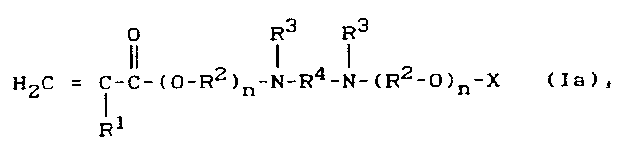 in der
R¹ Wasserstoff oder Methyl bedeutet,
R² und R⁴ zweiwertige aliphatische Reste mit ein bis drei Kohlenstoffatomen bedeuten, R³ Wasserstoff oder Formyl (-CO-H) bedeutet,
X Wasserstoff oder (Meth)acryloyl 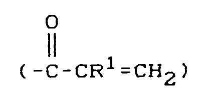 bedeutet und
n für 0 oder 1 steht,
mit der Maßgabe, daß für den Fall R³ gleich H n Null ist und gegebenenfalls Zusätze wie Initiatoren, Lösungsmittel und Füllstoffe als Adhäsivkomponente bei der Herstellung von Mitteln zur Behandlung kollagenhaltiger Materialien in der Medizin..

13. Verwendung nach Anspruch 12, nach einer Konditionierung des kollagenhaltigen Materials mit einer Flüssigkeit eines pH-Wertes von 0,1 bis 3,5.

14. Verwendung nach den Ansprüchen 12 und 13 als Klebemittel zur Befestigung von Zahnreparaturmaterialien am Zahn.

15. Verwendung nach Anspruch 12 als Klebemittel in Knochenzement.

## Patentansprüche (Patentansprüche für folgende(n) Vertragsstaat(en) : ES, GR)

1. Verfahren zur Herstellung von Zubereitungen zur Verwendung als Adhäsivkomponente zur Behandlung kollagenhaltiger Materialien, dadurch gekennzeichnet, daß man Alkandiyl-bis-carbonamide der Formel (I a) 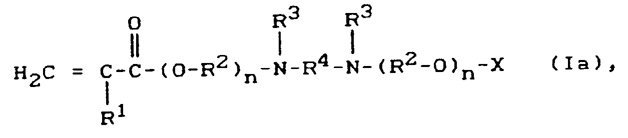 in der
R¹ Wasserstoff oder Methyl bedeutet,
R² und R⁴ zweiwertige aliphatische Reste mit ein bis drei Kohlenstoffatomen bedeuten, R³ Wasserstoff oder Formyl (-CO-H) bedeutet,
X Wasserstoff oder (Meth)acryloyl 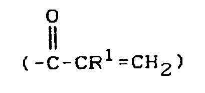 bedeutet und
n für 0 oder 1 steht, mit der Maßgabe, daß für den Fall R³ gleich H n Null ist und gegebenenfalls Initiatoren in einem Lösungsmittel mischt.

## Claims (Claims for the following Contracting State(s) : AT, BE, CH, DE, DK, FR, GB, IT, Ll, LU, NL, SE)

1. Alkanediyl-bis-carboxamides of the formula (I) 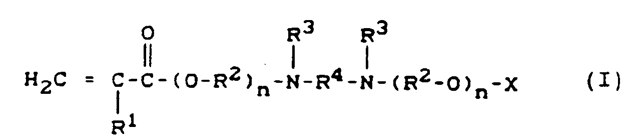 in which
R¹ denotes hydrogen or methyl,
R² and R⁴ denote divalent aliphatic radicals having two or three carbon atoms, R3 denotes formyl (-CO-H),
X denotes hydrogen or (meth)acryloyl 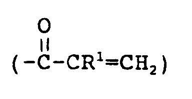 and
n represents 1.

2. Preparations containing alkanediyl-bis-carboxamides of the formula (la) 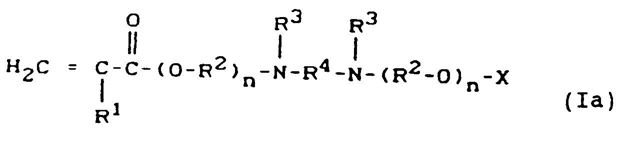 in which
R¹ denotes hydrogen or methyl,
R² and R⁴ denote divalent aliphatic radicals having up to three carbon atoms, R³ denotes hydrogen or formyl (-CO-H),
X denotes hydrogen or (meth)acryloyl 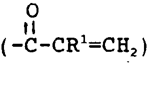 and
n represents 0 or 1,
with the proviso that for the case R³ equals H, n is zero, and, if appropriate, additives such as initiators, solvents and fillers as an adhesive component for the treatment of collagen-containing materials for use in medicine.

3. Preparation according to Claim 2, containing alkanediyl-bis-carboxamides (la) in which
R¹ denotes hydrogen or methyl,
R3 denotes hydrogen,
R4 denotes ethylene (-CH₂CH₂-),
X denotes hydrogen or (meth)acryloyl 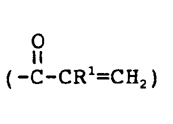 and
n represents zero.

4. Preparation according to Claim 2, containing alkanediyl-bis-carboxamides (la) in which
R¹ denotes hydrogen or methyl,
R2 and R⁴ denote ethylene (-CH₂CH₂-),
R3 denotes formyl (-CO-H),
X denotes hydrogen or (meth)acryloyl 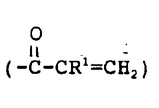 and
n represents 1.

5. Preparation according to Claims 2 to 4, characterized in that in that a free radical former from the series comprising the mono- or dicarbonyl compounds is employed as an initiator.

6. Preparation according to Claims 2 to 5, characterized in that the alkanediyl-bis-carboxamide and the initiator are dissolved in a solvent.

7. Preparation according to Claims 2 to 5, characterized in that a coactivator is added.

8. Preparation according to Claims 2 to 5, characterized in that a carbonyl compound is added as a further component.

9. Preparation according to Claims 2 to 8, characterized in that (meth)acrylic acid esters which can form crosslinkages are added as a further component.

10. Preparation according to Claims 2 to 9, characterized in that a filler is added as a further component.

11. Method for the production of preparations for-use as an adhesive component for the treatment of collagen-containing materials in medicine, characterized in that alkanediyl-bis-carboxamides of the formula (la) 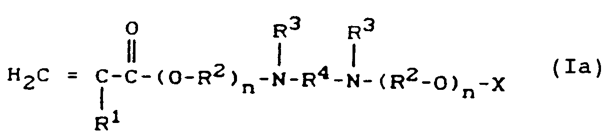 in which
R¹ denotes hydrogen or methyl,
R² and R⁴ denote divalent aliphatic radicals having one to three carbon atoms, R³ denotes hydrogen or formyl (-CO-H),
X denotes hydrogen or (meth)acryloyl 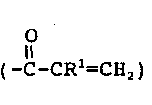 and
n represents 0 or 1,
with the proviso thatfor the case R³ equals H, n is zero, and, if appropriate, initiators are mixed in a solvent.

12. Use of preparations containing alkanediyl-bis-carboxamides of the formula (la) 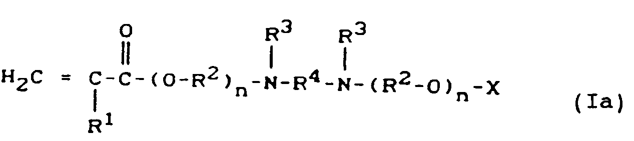 in which
R¹ denotes hydrogen or methyl,
R² and R⁴ denote divalent aliphatic radicals having one to three carbon atoms, R³ denotes hydrogen or formyl (-CO-H),
X denotes hydrogen or (meth)acryloyl 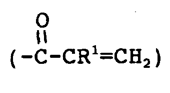 and
n represents 0 or 1,
with the proviso that for the case R³ equals H, n is zero, and, if appropriate, additives such as initiators, solvents and fillers as an adhesive component in the production of agents for the treatment of collagen-containing materials in medicine.

13. Use according to Claim 12, after conditioning of the collagen-containing material with a fluid of pH from 0.1 to 3.5.

14. Use according to Claims 12 and 13 as adhesives for attaching dental repair materials to the tooth.

15. Use according to Claim 12 as an adhesive in bone cement.

## Claims (Claims for the following Contracting State(s) : ES, GR)

1. Method for the production of preparations for use as an adhesive component for the treatment of collagen-containing materials, characterized in that alkanediyl-bis-carboxamides of the formula (la) 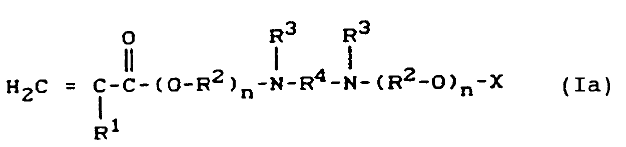 in which
R¹ denotes hydrogen or methyl,
R² and R⁴ denote divalent aliphatic radicals having one to three carbon atoms, R³ denotes hydrogen or formyl (-CO-H),
X denotes hydrogen or (meth)acryloyl 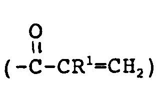 and
n represents 0 or 1,
with the proviso thatfor the case R³ equals H, n is zero, and, if appropriate, initiators are mixed in a solvent.

## Revendications (Revendications pour l'(les) Etat(s) contractant(s) suivant(s) : suivants : AT, BE, CH, DE, DK, FR, GB, IL, LI, LU, NL, SE)

1. Alcanediyl-bis-carboxamides de formule (I) 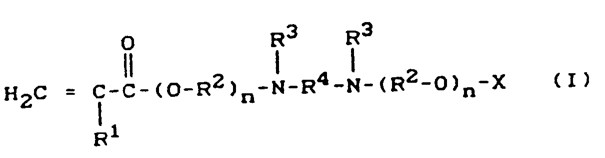 dans laquelle
R¹ représente l'hydrogène ou le groupe méthyle,
R² et R⁴ sont des restes aliphatiques divalents ayant deux ou trois atomes de carbone, R³ est un groupe formyle (-CO-H),
X est de l'hydrogène ou un groupe (méth)acryloyle 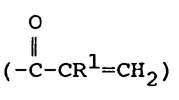 et
n a la valeur 1.

2. Préparations contenant des alcanediyl-bis-carboxamides de formule (la) 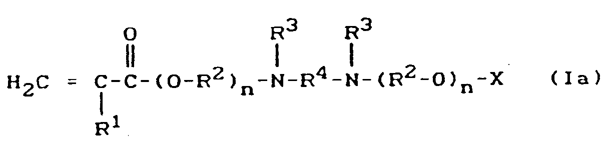 dans laquelle
R¹ représente l'hydrogène ou le groupe méthyle,
R² et R⁴ sont des restes aliphatiques divalents ayant jusqu'à 3 atomes de carbone, R³ est de l'hydrogène ou un groupe formyle (-CO-H),
X est de l'hydrogène ou un groupe (méth)acryloyle 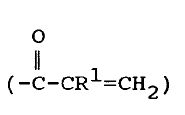
n a la valeur 0 ou 1,
sous réserve qu'au cas où R³ est égal à H, n soit égal à zéro, et le cas échéant des additifs tels que des initiateurs, des solvants et des charges, comme composant adhésif pour le traitement de matières contenant du collagène, destinées à être utilisées en médecine.

3. Préparation suivant la revendication 2, contenant des alcanediyl-bis-carboxamides (la), où
R¹ est de l'hydrogène ou un groupe méthyle,
R3 est de l'hydrogène,
R⁴ est un groupe éthylène (-CH₂CH₂-),
X est de l'hydrogène ou un groupe (méth)acryloyle 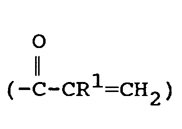 et
n est égal à zéro.

4. Préparation suivant la revendication 2, contenant des alcanediyl-bis-carboxamides (la), où
R¹ représente de l'hydrogène ou un groupe méthyle,
R² et R⁴ représentent un groupe éthylène (-CH₂CH₂-),
R³ est un groupe formyle (-CO-H),
X représente de l'hydrogène ou un groupe (méth)acryloyle 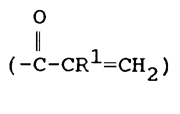 et
n est égal à 1.

5. Préparation suivant les revendications 2 à 4, caractérisée en ce qu'on utilise comme initiateur un générateur de radicaux de la série des composés monocarbonyliques ou dicarbonyliques.

6. Préparation suivant les revendications 2 à 5, caractérisée en ce que l'alcanediyl-bis-carboxamide et l'initiateur sont dissous dans un solvant.

7. Préparation suivant les revendications 2 à 5, caractérisée en ce qu'on ajoute un co-activateur.

8. Préparation suivant les revendications 2 à 5, caractérisée en ce qu'on ajoute comme autre composant un composé carbonylique.

9. Préparation suivant les revendications 2 à 8, caractérisée en ce qu'on ajoute comme autre composant des esters d'acides (méth)acryliques qui peuvent créer des réticulations.

10. Préparation suivant les revendications 2 à 9, caractérisée en ce qu'on ajoute une charge comme autre composant.

11. Procédé pour l'obtention de préparations destinées à être utilisées comme composant adhésif pour le traitement de matières contenant du collagène en médecine, caractérisé en ce qu'on mélange des alcanediyl-bis-carboxamides de formule (la) 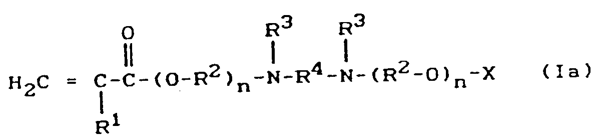 dans laquelle
R¹ représente l'hydrogène ou un groupe méthyle,
R² et R⁴ sont des restes aliphatiques divalents ayant 1 à 3 atomes de carbone, R³ est de l'hydrogène ou un groupe formyle (-CO-H),
X est de l'hydrogène ou un groupe méthy(acryloyle) 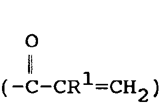 et
n a la valeur 0 ou 1,
sous réserve qu'au cas où R³ représente H, n soit égal à zéro et, le cas échéant, des initiateurs dans un solvant.

12. Utilisation de préparations contenant des alcanediyl-bis-carboxamides de formule (la) 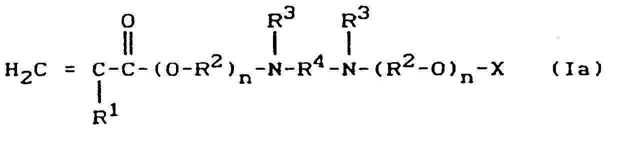 dans laquelle
R¹ représente l'hydrogène ou un groupe méthyle,
R² et R⁴ sont des restes aliphatiques divalents ayant 1 à 3 atomes de carbone, R³ est de l'hydrogène ou un groupe formyle (-CO-H),
X est de l'hydrogène ou un groupe (méth)acryloyle 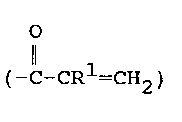 et
n a la valeur 0 ou 1,
sous réserve qu'au cas où R³ représente H, n soit égal à zéro et, le cas échéant, des additifs tels que des initiateurs, des solvants, des charges, comme composant adhésif dans la préparation de compositions pour le traitement de matières contenant du collagène en médecine.

13. Utilisation suivant la revendication 12, après un conditionnement de la matière contenant du collagène avec un liquide de pH ayant une valeur de 0,1 à 3,5.

14. Utilisation suivant les revendications 12 et 13 comme adhésif pour la fixation de matériaux de restauration dentaire à une dent.

15. Utilisation suivant la revendication 12, comme adhésif dans un ciment pour os.

## Revendications (Revendications pour l'(les) Etat(s) contractant(s) suivant(s) : ES, GR)

1. Procédé pour l'obtention de préparations destinées à être utilisées comme composant adhésif dans le traitement de matières contenant du collagène, caractérisé en ce qu'on mélange des alcanediyl-bis-carboxamides de formule (la) 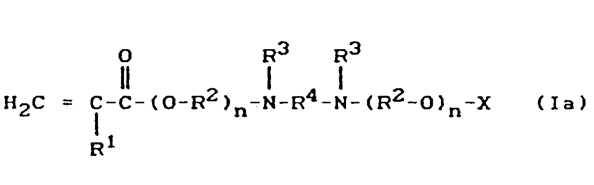 dans laquelle
R¹ représente l'hydrogène ou le groupe méthyle,
R² et R⁴ sont des restes aliphatiques divalents ayant un à trois atomes de carbone, R³ représente de l'hydrogène ou un groupe formyle (-CO-H),
X est de l'hydrogène ou un groupe (méth)acryloyle 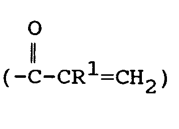 et
n a la valeur 0 ou 1,
sous réserve que lorsque R³ représente H, n soit égal à 0, et le cas échéant des initiateurs dans un solvant.
